# EUROPÄISCHE PATENTSCHRIFT

(11) **EP 0 987 241 B1**
(45) Veröffentlichungstag und Bekanntmachung des Hinweises auf die Patenterteilung: **04.12.2002**
(21) Anmeldenummer: 99115270.3
(22) Anmeldetag: 31.07.1999
(51) Int. Cl.: C07C 29/141, C07C 31/125, C07C 29/16

(54) **Verfahren zur selektiven Hydrierung von Hydroformylierungsgemischen**
Process for the selective hydrogenation of hydroformylation mixtures
Procédé pour l'hydrogénation sélective de mélanges d'hydroformylation

(30) Priorität: 16.09.1998 DE 19842370
(43) Veröffentlichungstag der Anmeldung: 22.03.2000
(73) Patentinhaber: Oxeno Olefinchemie GmbH, 45764 Marl (DE)
(72) Erfinder: Schuler, Joachim, Dr., 45772 Marl (DE); Kaizik, Alfred, Dr., 45772 Marl (DE); Scholz, Bernhard, Dr., 45768 Marl (DE); Büschken, Wilfried, Dr., 45721 Haltern (DE); Droste, Wilhelm, Dr., 45770 Marl (DE)

(56) Entgegenhaltungen:
- EP-A- 0 183 545
- EP-A- 0 326 674
- DE-A- 1 935 900
- GB-A- 1 157 698

## Beschreibung

Die Erfindung betrifft ein Verfahren zur selektiven Hydrierung von Hydroformylierungsgemischen, die bei der Herstellung höherer Oxo-Alkohole durch Hydroformylierung der entsprechenden Olefine anfallen. Selektiv ist die Hydrierung insofern, als die Aldehyde und bestimmte Nebenprodukte der Hydroformylierung zu den gewünschten Alkoholen hydriert werden, während die nicht umgesetzten Ausgangsolefine praktisch vollständig erhalten bleiben.

Höhere Alkohole, insbesondere solche mit 6 bis 25 C-Atomen, können bekanntlich durch katalytische Hydroformylierung (oder Oxo-Reaktion) der um ein C-Atom ärmeren Olefine und anschließende katalytische Hydrierung der Aldehyd- und Alkohol-haltigen Reaktionsgemische hergestellt werden. Sie werden vorwiegend als Edukte für die Herstellung von Weichmachern oder Detergentien verwendet.

Es ist bekannt, daß bei der katalytischen Hydroformylierung von Olefinen Reaktionsgemische entstehen, die außer den gewünschten Produkten, d.h. Aldehyden und den entsprechenden Alkoholen, in Abhängigkeit vom Katalysator und von den Reaktionsbedingungen neben nicht umgesetzten Olefinen Neben- und Folgeprodukte der Hydroformylierung, wie durch Hydrierung aus den Olefinen entstandene gesättigte Kohlenwasserstoffe, Wasser, Ester der gewünschten Alkohole (z.B. Formiate). Acetale aus den Zielprodukten Aldehyd und Alkohol, Enolether sowie andere Neben- oder Folgeprodukte enthalten können. Man kann die genannten Stoffe in Leichtsieder mit einem Siedepunkt unterhalb des Siedepunktes des Aldehyds und Hochsieder mit einem Siedepunkt oberhalb des Siedepunktes des Alkohols unterteilen. Bei der Hydrierung der Reaktionsgemische entstehen aus manchen der Nebenprodukte, wie Estern und Acetalen, die gewünschten Alkohole, was die Ausbeute verbessert. Andererseits ist es erwünscht, daß die nicht umgesetzten Olefine bei der Hydrierung erhalten bleiben, damit man sie nach Abtrennung aus dem Hydrierungsgemisch in die Hydroformylierungsreaktion zurückführen kann. Natürlich könnte man die Olefine schon von der Hydrierung des Hydroformylierungsgemisches durch Destillation von den Aldehyden und den Alkoholen abtrennen. Das würde jedoch einen zusätzlichen Verfahrensschritt bedeuten, denn das Hydroformylierungsgemisch muß nach der Hydrierung der Aldehyde ohnehin destilliert werden.

Die katalytische Hydrierung von Reaktionsgemischen, die durch Kobalt-katalysierte Hydroformylierung von Olefinen mit 2 bis 24 C-Atomen hergestellt wurden, ist in DE 35 42 595 beschrieben. Die Hydrierung wird zweistufig durchgeführt. In der ersten Stufe wird das Hydroformylierungsgemisch bei 150-230°C und einem Wasserstoffdruck von 10-350 bar mit 80-95%igem Umsatz an einem SiO₂-Trägerkatalysator hydriert, der 5-15 Gew.-% Nickel und 2-20 Gew-% Molybdän in Form von Molybdänoxid enthält. In der zweiten Stufe wird die Hydrierung bei 150-230°C und 10-350 bar Wasserstoffdruck an einem Katalysator zu Ende geführt, dessen aktive Masse aus 55-60 Gew.-% Kobalt, 15-20 Gew.-% Kupfer, 4-10 Gew.-% Mangan und 2-5 Gew.-% Molybdän in Form von Molybdänoxid sowie gegebenenfalls bis zu 10 Gew.-% aktivierenden Zusätzen besteht. Bei dem Verfahren werden die im Gemisch vorliegenden Formiate und Acetale zu den entsprechenden Alkoholen umgesetzt. Das Verfahren zielt offenbar nicht auf eine selektive Hydrierung unter Erhalt der Olefine ab, denn diese werden überhaupt nicht erwähnt. Nachteilig bei dem Verfahren ist weiterhin, daß die Hydrierung in zwei Stufen und bei hohen Drücken - nach dem Beispiel bei 250 bzw. 245 bar - erfolgt.

Nach US-A 5 399 793 werden für die Hydrierung von entkobalteten Reaktionsgemischen, wie sie bei der Hydroformylierung von C₅-C₁₂-Olefinen anfallen. Ni/Mo-Katalysatoren auf Al₂O₃ oder Al₂O₃·SiO₂ als Trägermaterialien verwendet. Das Gesamtverfahren umfaßt die folgenden Einzelschritte:
(a) Kobalt-katalysierte Hydroformylierung
(b) Entkobaltung des Reaktionsgemisches
(c) Hydrierung des rohen Reaktionsgemisches bei erhöhter Temperatur und unter erhöhtem Druck
(d) Gewinnung von Alkoholen mit sehr geringen Mangen an Aldehyden durch Destillation und
(e) Finishhydrierung der Alkohole.
Die Hydrierungen der Stufen (c) und/oder (e) können unter Verwendung eines bimetallischen, Phosphor-freien Ni/Mo-Hydrierkatalysators durchgeführt werden. Dieser Hydrierkatalysator erzeugt weniger hochsiedende Nebenprodukte als ein entsprechender Phosphor-haltiger Katalysator. In den Beispielen wird zwar die Anwesenheit von Leichtsiedern, nämlich Olefinen und Paraffinen erwähnt. Es finden sich jedoch keine Angaben über das Mengenverhältnis dieser Stoffe vor und nach der Hydrierung. Nachteilig ist auf jeden Fall, daß zur Herstellung eines spezifikationsgerechten, für die Herstellung von Weichmachern geeigneten Alkohols zwei Hydrierstufen notwendig sind und daß zumindest bei der Hydrierstufe (b) ein relativ hoher Druck von 1.000 psig (etwa 70 bar) erforderlich ist.

Es sind weiterhin Verfahren bekannt geworden, bei denen eine Verbindung, die eine Carbonylfunktion und eine olefinische Doppelbindung enthält, selektiv unter Erhalt der olefinischen Doppelbindung zum entsprechenden Alkohol katalytisch hydriert wird. So läßt sich nach der japanischen Patentanmeldung SHYO 57-110354 7-Octenal bei 70 bis 150°C selektiv zu 7-Octen-1-ol hydrieren, wobei man einen Chromoxid-Katalysator oder einen Katalysator verwendet, der aus mindestens zwei der Metalle Chrom, Kupfer und Zinn besteht. Dieses Verfahren hat jedoch den Nachteil, daß ein Lösungsmittel verwendet wird, das wieder abgetrennt werden muß. Weiterhin sind die erzielbaren Raum-Zeit-Ausbeuten im Temperatrurbereich von 70 bis 130°C für eine technische Anwendung zu gering. Bei höheren Temperaturen, bei denen die Hydriergeschwindigkeit größer ist, nimmt die Selektivität der Hydrierung aufgrund der unerwünschten Hydrierung zum gesättigten Alkohol rasch ab. Bei 140°C liegt die Hydrierselektivität zum ungesättigten Alkohol schon unter 95%.

Weiterhin läßt sich Citronellal unter Erhalt der olefinischen Doppelbindung zu Citronellol hydrieren. Dazu verwendet man nach Applied Catalysis, 25 (1986), 181-189, Rutheniumkatalysatoren und erzielt eine Ausbeute von bis zu 90%. Mit Cu/Cr-Katalysatoren wurde nach Iv.Akad.Nauk.Gruz. SSR, Ser. Khim. 16(4) (1990), 287-292, eine Ausbeute von 92% erzielt.

Andererseits ist bekannt, daß 2-Ethylhex-2-enal bei Verwendung eines Cu/Cr/Ni-Katalysators, der eine Alkalikomponente enthält, zu 2-Ethylhexanol hydriert werden kann (EP 0 326 674 A2). Dabei werden also sowohl die Carbonylfunktion als auch die olefinische Doppelbindung hydriert.

Der vorliegenden Erfindung lag die Aufgabe zugrunde. Reaktionsgemische der Hydroformylierung von C₅- bis C₂₄-Olefinen unter vergleichsweise milden Bedingungen und insbesondere niedrigen Drücken so selektiv zu hydrieren, daß die Aldehyde und bestimmte, neben Alkoholen und Aldehyden vorliegende Begleitstoffe, insbesondere die Formiate, möglichst weitgehend in die gewünschten Alkohole umgewandelt und die nicht umgesetzten Olefine möglichst wenig hydriert werden.

Diese Aufgabe wurde überraschend gelöst durch ein Verfahren zur selektiven Hydrierung von Reaktionsgemischen, die aus der Hydroformylierung von C₅- bis C₂₄-Olefinen stammen, mittels Wasserstoff an festen Katalysatoren bei erhöhter Temperatur und unter erhöhtem Druck, bei dem man als Katalysator einen Trägerkatalysator verwendet, der als aktive Komponenten Kupfer, Nickel und Chrom enthält und die Hydrierung kontinuierlich oder diskontinuierlich in flüssiger Phase durchführt.

Vorzugsweise verwendet man den aus EP 0 326 674 A2 bekannten Trägerkatalysator, der als aktive Komponenten Kupfer und Nickel in Konzentrationen von jeweils 0,3 bis 15 Gew.-%. Chrom in einer Konzentration von 0,05 bis 3,5 Gew.-% und eine Alkalimetallkomponente in einer Konzentration von 0.01 bis 1.6 Gew.-%, jeweils bezogen auf den Trägerkatalysator, enthält. Ein anderer vorteilhafter Trägerkatalysator enthält Kupfer, Nickel und Chrom in den angegebenen Mengen, aber keine Alkalimetallkomponente.

Das erfindungsgemäße Verfahren bietet eine Reihe von Vorteilen. Die Aldehyde in den Hydroformylierungsgemischen werden bei Umsätzen über 98% in einer Selektivität von über 99% in nur einer Hydrierstufe zu den entsprechenden Alkoholen hydriert. Die Ester und insbesondere die Formiate sowie die Acetale werden ebenfalls in die gewünschten Alkohole umgewandelt. Die im Gemisch enthaltenen Ausgangsolefine bleiben überraschenderweise weit überwiegend unverändert, obwohl dieselben Katalysatoren unter vergleichbaren Bedingungen die olefinische Doppelbindung im 2-Ethylhex-2-enal praktisch quantitativ hydrieren. Dengegenüber werden bei dem erfindungsgemäßen Verfahren weniger als 5 % der Olefine des Edukts zu Kohlenwasserstoffen hydriert. Die Hydrierung kann im Niederdruckbereich von unter 25 bar und mit hohen Raum-Zeit-Ausbeuten durchgeführt werden.

Aus DE 193 59 00 ist ein Verfahren zur Gasphasenhydrierung von Aldehyden und Ketonen aus kohlenmonoxidreichen Gasströmen bekannt. EP 0 183 545 offenbart ein Hydroformylierungsverfahren für Olefine, bei dem die Hydroformylierungsprodukte zunächst hydriert, in Hoch- und Niedrigsieder aufgetrennt und die Hochsieder anschließend einem katalytischen Steamcracking unterzogen werden.

Die Edukte für die Hydroformylierung sind Monoolefine mit 5 bis 24 Kohlenstoffatomen und end- oder mittelständiger C-C-Doppelbindung oder deren Gemische, wie 1- oder 2-Penten, 2-Methyl-1-buten, 1-, 2-oder 3-Hexen, das bei der Dimerisierung von Propen anfallende isomere C₆-Olefingemisch (Dipropen), 3-Methyl-1-hexen, 1-Octen, das bei der Dimerisierung von Butenen anfallende isomere C₈-Olefingemisch (Dibuten), 1-Nonen, 2-, 3- oder 4-Methyl-1-octen, das bei der Trimerisierung von Propen anfallende isomere C₉-Olefingemisch (Tripropen), 1-, 2- oder 3-Decen, 2-Ethyl-1-octen, 1-Dodecen, das bei der Tetramerisierung von Propen oder der Trimerisierung von Butenen anfallende isomere C₁₂-Olefingemisch (Tetrapropen oder Tributen), 1-Tetradecen, 1- oder 2-Hexadecen, bei der Tetramerisierung von Butenen anfallende C₁₆-Olefingemische (Tetrabuten) sowie durch Cooligomerisierung von Olefinen mit unterschiedlichen C-Zahlen (bevorzugt 2 bis 4) hergestellte Olefingemische, gegebenenfalls nach destillativer Trennung in Fraktionen mit gleicher oder ähnlicher C-Zahl. Vorzugsweise werden Gemische hydriert, die bei der Hydroformylierung von C₈-, C₉-, C₁₂oder C₁₆-Olefingemischen entstehen.

Die Olefine werden in üblicher Weise hydroformyliert und ergeben dann die Edukte für das erfindungsgemäße Hydrierverfahren. Man arbeitet also mit Rhodium- oder vorzugsweise mit Kobaltkatalysatoren sowie mit oder ohne Komplex-stabilisierende Zusätzen, wie organischen Phosphinen oder Phosphiten. Die Temperaturen und die Drücke können, je nach Katalysator und Olefin, in weiten Grenzen variieren. Eine Beschreibung der Hydroformylierung von Olefinen findet sich z.B. bei J.Falbe. New Syntheses with Carbon Monoxide. Springer-Verlag. Heidelberg-New York, 1980, Seiten 99ff, sowie bei Kirk-Othmer, Encyclopedia of Chemical Technology, Band 17, 4. Auflage, John Wiley & Sons, Seiten 902-919 (1996).

Die Reaktionsgemische der Hydroformylierung werden zweckmäßig zunächst vom Katalysator befreit. Wenn ein Kobaltkatalysator verwendet wurde, kann dies durch Druckentlastung, Abtrennung der wäßrigen Katalysatorphase, Oxidation der im Hydroformylierungsgemisch verbliebenen Kobaltcarbonylverbindungen mit Luft oder Sauerstoff und Auswaschen der entstandenen Kobaltverbindungen mit Wasser oder wäßriger Säure geschehen. Entkobaltungsverfahren sind gut bekannt, siehe z.B. J.Falbe, a.a.0., Kirk-Othmer, a.a.0., 164, 175, BASF-Verfahren; sowie EP-0 850 905 A1.

Wenn eine Rhodiumverbindung als Hydroformylierungskatalysator diente, kann man sie mittels Dünnschichtverdampfung als Destillationsrückstand vom Hydroformylierungsgemisch abtrennen.

Die zweckmäßig vom Hydroformylierungskatalysator befreiten Reaktionsgemische der Hydroformylierung enthalten im allgemeinen 3-40 Gew.-%, meistens 5-30 Gew.-% Leichtsieder, hauptsächlich Olefine, daneben die entsprechenden gesättigten Kohlenwasserstoffe sowie Wasser, 30-90 Gew.-% Aldehyde, 5-60 Gew.-% Alkohole, bis zu 10 Gew.-% Formiate dieser Alkohole und 5 bis 15 Gew.-% Hochsieder. Es sei jedoch betont, daß das erfindungsgemäße Verfahren auch mit Hydroformylierungsgemischen ausgeführt werden kann, deren Zusammensetzung in dieser und bzw. oder jener Beziehung nicht diesen Angaben entspricht.

Bevorzugte Katalysatoren, an denen die Hydroformylierungsgemische hydriert werden, enthalten jeweils 0,3-15 Gew.-% Kupfer und Nickel sowie als Aktivatoren 0,05-3,5 Gew.-% Chrom und vorteilhaft 0,01-1,6 Gew.-%. vorzugsweise 0,02-1,2 Gew.-% einer Alkalimetallkomponente auf einem Trägermaterial, vorzugsweise Aluminumoxid oder Siliciumdioxid. Die Mengenangaben beziehen sich auf den wie folgt beschrieben hergestellten, noch nicht reduzierten Katalysator. Die Alkalimetallkomponente kann, wie erwähnt, auch fehlen.

Die genannten Katalysatorkomponenten können homogen in den Poren eines Trägermaterials verteilt oder in dessen Randzonen angereichert sein. Im ersteren Fall setzt man eine wässerige Lösung an, die die Komponenten in Form von Metallsalzen als Katalysatorvorläufer enthält und deren Volumen zweckmäßig ungefähr dem 0,8-fachen des Porenvolumens des Trägermaterials entspricht. Als Kupfer-, Nickel- bzw. Chromsalze verwendet man vorteilhaft solche, die beim Erhitzen in Oxide übergehen, wie Nitrate und Acetate. Wenn der Katalysator eine Alkalikomponente enthalten soll, kann diese zusammen mit Chrom in Form von Alkalichromat oder -dichromat, insbesondere als Natriumchromat oder -dichromat eingebracht werden. Die Konzentration der Metallsalze in der Lösung hängt von der gewünschten Konzentration der jeweiligen Komponente im fertigen Katalysator ab. Die Metallsalzlösung wird dann auf das in einer Dragiertrommel befindliche, nicht vorerhitzte Trägermaterial aufgesprüht und dringt in dessen Poren ein. Danach wird der Katalysator getrocknet.

Ist ein Katalysator mit Komponenten erwünscht, die in den Randzonen eines porösen oder eines mehr oder minder porenfreien Trägermaterials angereichert sind, so kann man die Metallsalzlösung auf ein vorerhitztes Trägermaterial aufsprühen und das Trägermaterial während des Aufsprühens weiter erhitzen, so daß das Wasser verdampft und die Katalysatorkomponenten im wesentlichen auf der Oberfläche des Trägermaterials fixiert werden.

Nach dem Aufbringen der Katalysatorkomponenten werden die Katalysatoren calciniert, d.h. je nach dem verwendeten Katalysatorvorläufer auf Temperaturen von 200-400°C erhitzt, wodurch die Katalysatorvorläufer in den oxidischen Zustand überführt werden. Anschließend wird der Katalysator mit Wasserstoff bei den genannten Hydriertemperaturen reduziert. Die Reduktion kann gleich nach der Herstellung des Katalysators oder zweckmäßig erst in dem Hydrierungsreaktor erfolgen.

Die Katalysatoren werden vorteilhaft in einer Form eingesetzt, in der sie einen geringen Strömungswiderstand bieten, z.B. in Form von Granalien, Pellets oder Formkörpern, wie Tabletten, Zylindern, Strangextrudaten oder Ringen. Sie werden zweckmäßig vor ihrem Einsatz durch Erhitzen im Wasserstoffstrom, beispielsweise auf 150 bis 250°C, aktiviert, sofern sie nicht im Reaktor reduziert wurden.

Die erfindungsgemäße Hydrierung wird kontinuierlich oder diskontinuierlich in flüssiger Phase durchgeführt werden. Die Hydrierung in flüssiger Phase wird angewendet, weil ein Gasphasenverfahren wegen der notwendigen Kreisführung großer Gasvolumina einen höheren Energieaufwand erfordern würde. Hierzu kommt, daß die Verdampfung der Aldehyde mit steigender C-Zahl mehr und mehr Energie erfordert und zudem die Eduktbeladung des Reduktionsgases abnimmt, so daß ein Gasphasenverfahren bei Aldehyden mit einer C-Zahl größer als etwa 8 kaum noch wirtschaftlich betrieben werden kann.

Für die Flüssigphasenhydrierung können unterschiedliche Verfahrensvarianten gewählt werden. Sie kann adiabatisch oder praktisch isotherm. d.h. mit einem Temperaturanstieg von <10°C, ein- oder zweistufig durchgeführt werden. Im letzteren Fall kann man beide Reaktoren, zweckmäßig Rohrreaktoren, adiabatisch oder praktisch isotherm oder den einen adiabatisch und den anderen praktisch isotherm betreiben. Weiterhin ist es möglich, die Hydroformylierungsgemische im geraden Durchgang oder mit Produktrückführung zu hydrieren. Die Reaktoren können als Gleichstromreaktoren mit Rieselbett (trickle flow) oder bevorzugt mit hohen Flüssigkeitsbelastungen (pulse flow) betrieben werden. Im Interesse einer hohen Raum-Zeit-Ausbeute werden die Reaktoren vorzugsweise mit hohen Flüssigkeitsbelastungen von 5-100 m³, insbesondere von 15-50 m³ pro m² Querschnitt des leeren Reaktors und Stunde betrieben. Wird ein Reaktor isotherm und im geraden Durchgang betrieben, so kann die spezifische Katalysatorbelastung (LHSV) Werte zwischen 0.1 und 10 h⁻¹, vorzugsweise zwischen 0.5 und 5 h⁻¹ annehmen.

Die Flüssigphasenhydrierung wird im allgemeinen unter einem Gesamtdruck von 5 bis 30 bar, insbesondere zwischen 15 und 25 bar durchgeführt. Die Hydrierung in der Gasphase kann auch bei niedrigeren Drükken durchgeführt werden, mit entsprechend größeren Gasvolumina. Die Reaktionstemperaturen liegen bei Hydrierungen in flüssiger oder gasförmiger Phase in der Regel zwischen 120 und 220°C, insbesondere zwischen 140 und 180°C.

Nach der Hydrierung werden die Reaktionsgemische durch Destillation aufgearbeitet. Dies geschieht zweckmäßig unter vermindertem Druck. z.B. bei einem absoluten Druck von 400-900 mbar. Die Olefine können in die Hydroformylierung zurückgeführt werden.

Die folgenden Beispiele sollen die Erfindung weiter erläutern, nicht aber ihren Anwendungsbereich beschränken, der sich aus den Patentansprüchen ergibt.

### Beispiel 1

### Hydrierung von C₁₃-Aldehyden in der Flüssigphase

Ein Liter eines durch Dünnschichtverdampfung von Katalysator befreiten Reaktionsaustrags der Rh-katalysierten Hydroformylierung eines C₁₂-Olefingemisches (Tributen) wurde in einer Kreislaufapparatur bei 175°C und einem Gesamtdruck von 20 bar mit Wasserstoff an 100 g Katalysator hydriert. Der Katalysator mit Aluminiumoxid als Trägermaterial enthielt vor der Aktivierung mit Wasserstoff (8 h bei 200°C)
12,1 Gew.-% Cu
3,0 Gew.-% Ni
und 2,5 Gew.-% Cr

Der Katalysator lag in Form von Strangextrudaten mit einer Schüttdichte von 0,67 kg/l vor. Die Edukt- und Produktanalysen gehen aus der folgenden Tabelle 1 hervor.

**Tabelle 1**

| (Stoffmengen in Gew.-%; 1/LHSV in l_{Kat}·h/l_{edukt}) | | | | | | | | |
|---|---|---|---|---|---|---|---|---|
| | 1/LHSV | Olefine | Paraffine | Aldehyde | Alkohole | Ester | Hochsieder | Wasser |
| Edukt | 0 | 14,8 | 1,3 | 76,5 | 6,4 | 0 | 1,0 | 0 |
| Produkt | 1,75 | 14,3 | 1,8 | 0,5 | 82,1 | 0 | 1,3 | 0 |

Man erkennt, daß mehr als 99% der Aldehyde. aber nur ca. 3% der Olefine hydriert wurden.

### Beispiel 2

### Hydrierung von C₉-Aldehyden in der Flüssigphase

Ein Liter eines entkobaltierten Reaktionsaustrags der Kobalt-katalysierten Hydroformylierung eines C₈-Olefingemisches (Dibuten) wurde in einer Kreislaufapparatur bei 170°C mit Wasserstoff hydriert, wobei der Gesamtdruck 20 bar betrug. Es wurden 100 g des Katalysators des Beispiels 1 eingesetzt, die Flüssigkeitsbelastung betrug 35 m³ pro m² Reaktorquerschnitt (Leerrohr) und Stunde.

Die Edukt- und Produktanalysen gehen aus der folgenden Tabelle 2 hervor.

**Tabelle 2**

| (Stoffmengen in Gew.-%, mit Ausnahme des Wassers wasserfrei gerechnet); 1/LHSV in l_{Kat}·h/l_{edukt}) | | | | | | | | | |
|---|---|---|---|---|---|---|---|---|---|
| | 1/LHSV | Olefine | Paraffine | Aldehyde | Alkohole | Ester | Methanol | Hochsieder | Wasser |
| Edukt | 0 | 7,6 | 4,4 | 47,2 | 32,1 | 4,5 | 0 | 4,2 | 2 |
| Produkt | 0,65 | 7,3 | 4,7 | 0,3 | 82,5 | 0,1 | 0,8 | 4,3 | 2 |

Man erkennt, daß wiederum mehr als 99% der Aldehyde, aber nur ca. 4% der Olefine hydriert wurden.

### Beispiel 3

### Hydrierung von C₉-Aldehyden in der Flüssigphase

Ein Liter eines durch Dünnschichtverdampfung von Katalysator befreiten Reaktionsaustrags der Rh-katalysierten Hydroformylierung eines C₈-Olefingemisches (Dibuten) wurde in einer Kreislaufapparatur bei 170°C mit Wasserstoff hydriert, wobei der Gesamtdruck 20 bar betrug. Es wurden 100 g des Katalysators des Beispiels 1 eingesetzt, die Flüssigkeitsbelastung betrug wiederum 35 m³ pro m² Reaktorquerschnitt und Stunde.

Die Edukt- und Produktanalysen gehen aus der folgenden Tabelle 3 hervor.

**Tabelle 3**

| (Stoffmengen in Gew.-%; 1/LHSV in l_{Kat}·h/l_{edukt}) | | | | | | | | |
|---|---|---|---|---|---|---|---|---|
| | 1/LHSV | Olefine | Paraffine | Aldehyde | Alko hole | Ester | Hochsieder | Wasser |
| Edukt | 0 | 5,4 | 0,6 | 87,9 | 4,1 | 0 | 2,0 | 0 |
| Produkt | 0,55 | 5,2 | 0,8 | 0,3 | 91,6 | 0 | 2,1 | 0 |

Man erkennt, daß mehr als 99% der Aldehyde, aber nur ca. 4% der Olefine hydriert wurden.

## Patentansprüche

1. Verfahren zur selektiven Hydrierung von Reaktionsgemischen aus der Hydroformylierung von C₅- bis C₂₄-Olefinen mittels Wasserstoff an festen Katalysatoren bei erhöhter Temperatur und unter erhöhtem Druck, **dadurch gekennzeichnet, daß** man einen Trägerkatalysator verwendet, der als aktive Komponenten Kupfer, Nickel und Chrom enthält und die Hydrierung kontinuierlich oder diskontinuierlich in flüssiger Phase durchführt.

2. Verfahren nach Anspruch 1, **dadurch gekennzeichnet, daß** man einen Trägerkatalysator verwendet, der als aktive Komponenten Kupfer und Nickel in Konzentrationen von jeweils 0,3 bis 15 Gew.-%, Chrom in einer Konzentration von 0,05 bis 3,5 Gew.-% und eine Alkalimetallkomponente in einer Konzentration von 0,01 bis 1,6 Gew.-%, jeweils bezogen auf den Trägerkatalysator, enthält.

3. Verfahren nach Anspruch 2, **dadurch gekennzeichnet, daß** die Konzentration der Alkalikomponente 0,2-1,2 Gew.-% beträgt.

4. Verfahren nach Anspruch 2, **dadurch gekennzeichnet, daß** der Trägerkatalysator keine Alkalimetallkomponente enthält.

5. Verfahren nach einem der Ansprüche 1 bis 4, **dadurch gekennzeichnet. daß** das Trägermaterial Siliciumdioxid oder Aluminumoxid ist.

6. Verfahren nach einem Ansprüche 1 bis 5, **dadurch gekennzeichnet, daß** die genannten Katalysatorkomponenten homogen in den Poren des Trägermaterials verteilt sind.

7. Verfahren nach einem Ansprüche 1 bis 5, **dadurch gekennzeichnet, daß** die genannten Katalysatorkomponenten homogen in den Randzonen des Trägermaterials angereichert sind.

8. Verfahren nach einem Ansprüche 1 bis 7, **dadurch gekennzeichnet**, man Gemische hydriert, die bei der Hydroformylierung von C₈-, C₉-, C¹²- oder C₁₆-Olefingemischen entstehen.

9. Verfahren nach einem der Ansprüche 1 bis 8, **dadurch gekennzeichnet, daß** man die Hydrierung in flüssiger Phase unter einem Gesamtdruck von 5 bis 30 bar durchführt.

10. Verfahren nach Anspruch 9, **dadurch gekennzeichnet, daß** der Gesamtdruck 15 bis 25 bar beträgt.

11. Verfahren nach einem der Ansprüche 1 bis 10, **dadurch gekennzeichnet, daß** man die Hydrierung bei 120 bis 220°C durchführt.

12. Verfahren nach Anspruch 11, **dadurch gekennzeichnet, daß** die Temperatur 140 bis 180°C beträgt.

13. Verfahren nach einem der Ansprüche 1 bis 12, **dadurch gekennzeichnet, daß** man die Hydrierung in flüssiger Phase und mit Flüssigkeitsbelastungen von 5-100 m³ pro m² Querschnitt des leeren Reaktors und Stunde durchführt.

14. Verfahren nach Anspruch 13, **dadurch gekennzeichnet, daß** die Flüssigkeitsbelastung 15-50 m³ pro m² Querschnitt des leeren Reaktors und Stunde beträgt.

15. Verfahren nach einem der Ansprüche 1 bis 14 **dadurch gekennzeichnet, daß** man das Hydrierungsgemisch durch Destillation trennt und die Olefine in die Hydroformylierung zurückführt.

## Claims

1. A process for the selective hydrogenation of reaction mixtures from the hydroformylation of C₅ to C₂₄ olefins using hydrogen on a solid catalyst at elevated temperature and at elevated pressure, **characterized in that** a supported catalyst is used which, as active components, comprises copper, nickel and chromium, and the hydrogenation is carried out continuously or batchwise in the liquid phase.

2. A process according to claim 1, **characterized in that** use is made of a supported catalyst which, as active components, comprises copper and nickel at concentrations of in each case from 0.3 to 15% by weight, chromium at a concentration of from 0.05 to 3.5% by weight and an alkali metal component at a concentration of from 0.01 to 1.6% by weight, in each case based on the supported catalyst.

3. A process according to claim 2, **characterized in that** the concentration of the alkali metal component is 0.2-1.2% by weight.

4. A process according to claim 2, **characterized in that** the supported catalyst does not comprise an alkali metal component.

5. A process according to any one of claims 1 to 4, **characterized in that** the support material is silicon dioxide or aluminium oxide.

6. A process according to any one of claims 1 to 5, **characterized in that** the said catalyst components are homogeneously distributed in the pores of the support material.

7. A process according to any one of claims 1 to 5, **characterized in that** the said catalyst components are homogeneously enriched in the edge zones of the support material.

8. A process according to any one of claims 1 to 7, **characterized in that** mixtures which are produced in the hydroformylation of C₈, C₉, C₁₂ or C₁₆ olefin mixtures are hydrogenated.

9. A process according to any one of claims 1 to 8, **characterized in that** the hydrogenation is carried out in the liquid phase at an overall pressure of from 5 to 30 bar.

10. A process according to claim 9, **characterized in that** the overall pressure is from 15 to 25 bar.

11. A process according to any one of claims 1 to 10, **characterized in that** the hydrogenation is carried out at from 120 to 220°C.

12. A process according to claim 11, **characterized in that** the temperature is from 140 to 180°C.

13. A process according to any one of claims 1 to 12, **characterized in that** the hydrogenation is carried out in the liquid phase and at liquid loadings of 5-100 m³ per m² of cross section of the empty reactor and hour.

14. A process according to claim 13, **characterized in that** the liquid loading is 15-50 m³ per m² of cross section of the empty reactor and hour.

15. A process according to any one of claims 1 to 14, **characterized in that** the hydrogenation mixture is separated by distillation and the olefins are recirculated to the hydroformylation.

## Revendications

1. Procédé d'hydrogénation sélective de mélanges réactionnels à partir de l'hydroformylation d'oléfines en C₅ à C₂₄ à l'aide d'hydrogène sur des catalyseurs solides à température accrue et sous pression accrue,
**caractérisé en ce qu'**
on utilise un catalyseur sur support qui contient comme composants actifs du cuivre, du nickel et du chrome, et on effectue l'hydrogénation en continu ou d'une manière discontinue en phase liquide.

2. Procédé selon la revendication 1,
**caractérisé en ce qu'**
on utilise un catalyseur sur support qui contient comme composants actifs du cuivre et du nickel à des concentrations de respectivement 0,3 à 15 % en poids, du chrome à une concentration de 0,05 à 3,5 % en poids, et un composant de métal alcalin à une concentration de 0,01 à 1,6 % en poids, à chaque fois rapporté au catalyseur sur support.

3. Procédé selon la revendication 2,
**caractérisé en ce que**
la concentration des composants alcalins est de à 0,2 à 1,2 % en poids.

4. Procédé selon la revendication 2,
**caractérisé en ce que**
le catalyseur sur support ne contient pas de composant de métal alcalin.

5. Procédé selon l'une des revendications 1 à 4,
**caractérisé en ce que**
le matériau de support est du dioxyde de silicium ou de l'oxyde d'aluminium.

6. Procédé selon l'une des revendications 1 à 5,
**caractérisé en ce que**
les composants de catalyseur cités sont répartis d'une manière homogène dans les pores du matériau de support.

7. Procédé selon l'une des revendications 1 à 5,
**caractérisé en ce que**
les composants du catalyseur cités sont enrichis d'une manière homogène dans les zones de bordure du matériau de support.

8. Procédé selon l'une des revendications 1 à 7,
**caractérisé en ce qu'**
on hydrogène des mélanges qui se forment au cours de l'hydroformylation de mélanges d'oléfines en C₈, C₉, C₁₂ ou C₁₆.

9. Procédé selon l'une des revendications 1 à 8,
**caractérisé en ce qu'**
on effectue l'hydrogénation en phase liquide sous une pression totale de 5 à 30 bars.

10. Procédé selon la revendication 9,
**caractérisé en ce que**
la pression totale est de 15 à 25 bars.

11. Procédé selon l'une des revendications 1 à 10,
**caractérisé en ce qu'**
on effectue l'hydrogénation à de 120 à 220°C.

12. Procédé selon la revendication 11,
**caractérisé en ce que**
la température atteint de 140 à 180°C.

13. Procédé selon l'une des revendications 1 à 12,
**caractérisé en ce qu'**
on effectue l'hydrogénation en phase liquide et avec des contraintes de liquide de 5 à 100 m³ par m² de section du réacteur vide et par heure.

14. Procédé selon la revendication 13,
**caractérisé en ce que**
la contrainte de liquide s'élève à de 15 à 50 m³ par m² de section du réacteur vide et par heure.

15. Procédé selon l'une des revendications 1 à 14,
**caractérisé en ce qu'**
on sépare le mélange d'hydrogénation par distillation et ou recycle les oléfines dans l'hydroformylation.
